# FASCICULE DE BREVET EUROPEEN

(11) **EP 0 779 080 B1**
(45) Date de publication et mention de la délivrance du brevet: **02.05.2003**
(21) Numéro de dépôt: 96402645.4
(22) Date de dépôt: 06.12.1996
(51) Int. Cl.: A61N 1/05

(54) **Sondes à barbes d'ancrage pliables pour dispositif médical implanté, notamment pour stimulateur cardiaque**
Stimulationsleitung mit ausfaltbarer Verankerungsvorrichtung für ein medizinisches, implantierbares Gerät, insbesondere für einen Herzschrittmacher
Pacing lead with folding anchoring tines for an implantable medical device, especially for a heart pacemaker

(30) Priorité: 12.12.1995 FR 9514680
(43) Date de publication de la demande: 18.06.1997
(73) Titulaire: ELA MEDICAL, F-92120 Montrouge (FR)
(72) Inventeur: Ollivier, Jean-François, 78280 Guyancourt (FR); Rottembourg, Jean-Loup, 78350 Les Loges en Josas (FR)
(74) Mandataire: Dupuis-Latour, Dominique

(56) Documents cités:
- EP-A- 0 546 414
- DE-A- 3 300 050
- US-A- 4 419 819
- US-A- 4 913 164
- US-A- 5 344 439

## Description

L'invention concerne une sonde pour dispositif médical implanté, notamment pour stimulateur cardiaque.

Ces sondes, notamment les sondes endocavitaires pour stimulateur cardiaque, comportent des systèmes de rétention montés sur leur extrémité distale, à proximité de l'électrode de stimulation proprement dite (ou des électrodes, dans le cas d'une sonde bipolaire). Divers systèmes de rétention ou d'ancrage ont été proposés, notamment des fixations en forme de crochets ou "barbes" qui viennent se coincer d'eux-mêmes entre les trabéculations endocardiques.

Le US-A-4 236 529 décrit une telle sonde, du type "fixation passive", c'est-à-dire dans laquelle les barbes viennent se loger d'elles-mêmes en place grâce à leur élasticité, leur forme inclinée assurant un effet anti-retour sans autre manoeuvre particulière.

Il a été également proposé des sondes à actuateur mécanique, comme par exemple dans les US-A-4 945 922, US-A-4 957 118 ou EP-A-0 546 414 dans lesquels les barbes, initialement repliées contre le corps de la sonde, sont déployées en manoeuvrant un mandrin coaxial inséré dans la sonde au moment de l'implantation et dont la rotation vient faire saillir les barbes une fois l'extrémité distale de la sonde positionnée à l'endroit voulu.

L'intérêt de ces dernières fixations tient au fait qu'elles sont généralement réversibles, de manière à permettre une explantation de la sonde par exemple après repliement des barbes par une manoeuvre inverse d'un mandrin inséré dans le canal axial central de la sonde. En revanche, ces sondes sont à la fois plus encombrantes (le diamètre du corps est plus important car il faut prévoir un canal axial) et de structure plus complexe ― donc plus coûteuse ― du fait de la nécessité de prévoir un système mécanique de transmission du mouvement de rotation du mandrin et de conversion de ce mouvement de rotation en un mouvement de déploiement des barbes articulées.

L'un des buts de la présente invention est de proposer une sonde qui puisse être mise en place sans actuateur du système d'ancrage. Un autre but de l'invention est de proposer une telle sonde qui soit extractible sans risque d'arrachement des trabéculations ou des tissus fibreux qui auraient pu se former autour de l'extrémité de la sonde.

La sonde de la présente invention cumule ainsi les avantages de faible diamètre, structure simple et donc peu coûteuse, facilité d'implantation sans recours à une instrumentation particulière et possibilité d'extraction sans endommagement des tissus environnants. Elle permet, comme on l'expliquera plus bas, d'utiliser un corps de sonde étanche et indépendant du système de déploiement des barbes.

À cet effet, la sonde selon l'invention comporte d'une part, un corps cylindrique portant l'électrode ou les électrodes de la sonde et, d'autre part, un manchon amovible coiffant le corps d'électrode. Le manchon comporte une bague de retenue du manchon sur le corps, le montage de cette bague sur le corps étant amovible de manière à permettre la dissociation ultérieure du manchon d'avec le corps par traction axiale de ce dernier, et des moyens déformables s'étendant à partir de la bague de retenue et susceptibles de prendre deux configurations stables. Le passage de la première à la seconde configuration résulte d'une sollicitation extérieure exercée axialement sur le manchon, et les moyens déformables ne font essentiellement pas saillie radialement dans la première configuration, de manière à permettre l'introduction en place de la sonde, et faisant essentiellement saillie radialement dans la seconde configuration de manière à former des barbes d'ancrage.

De préférence, les barbes d'ancrage des moyens déformables sont reliées entre elles par un élément mobile entourant le corps et coulissant le long de celui-ci entre une position initiale et une position finale, ces deux positions correspondant respectivement aux deux configurations stables des moyens déformables, ces moyens déformables formant moyens de liaison entre la bague de retenue et l'élément mobile.

Dans un premier mode de réalisation, l'élément mobile est une jupe placée en position distale par rapport à la bague, cette jupe faisant axialement saillie du corps en position initiale et n'en faisant pas saillie en position finale, et le passage de la première à la seconde configuration résultant de la sollicitation extérieure exercée axialement dans le sens proximal sur l'extrémité distale de la jupe lors de l'accostage de celle-ci avec une paroi contre laquelle doit venir en contact l'électrode.

Avantageusement, il est alors prévu des moyens pour augmenter le frottement entre corps et jupe au voisinage de la position finale, notamment lorsque le corps présente un diamètre extérieur accru et/ou la jupe présente un diamètre intérieur accru, ou encore lorsque le corps présente des reliefs sur sa surface extérieure et/ou la jupe présente des reliefs sur sa surface intérieure.

Le corps peut présenter un épaulement de butée en arrière de la position finale, et il peut en outre être prévu sur la face frontale du corps une vis d'ancrage, cette vis étant recouverte par la jupe en position initiale et en faisant saillie axialement en position finale. Cette dernière configuration protège les tissus de traumatismes susceptibles d'être causés par la vis d'ancrage lors de l'introduction de la sonde jusqu'à son placement dans une cavité cardiaque.

Dans un second mode de réalisation, l'élément mobile est un anneau placé en position proximale par rapport à la bague, et le passage de la première à la seconde configuration résulte de la sollicitation extérieure exercée axialement dans le sens distal sur l'anneau par manipulation d'une gaine enfilée sur le corps. Dans ce cas, le corps porte avantageusement une gorge périphérique de blocage recevant et immobilisant l'anneau en fin de course.

Dans tous les cas, les moyens de liaison comprennent de préférence une pluralité de ponts en un matériau élastiquement déformable, les ponts ayant notamment en section un profil concave, cette concavité s'inversant entre la première et la seconde configuration.

Par ailleurs, la bague de retenue a avantageusement une forme essentiellement torique et le corps cylindrique possède une gorge périphérique de section circulaire recevant cette bague torique, gorge et tore ayant des diamètres homologues sensiblement identiques de manière à permettre en tout point le pivotement local de la bague dans la gorge.

On va maintenant décrire des exemples de mise en oeuvre de l'invention.

La figure 1 est une vue perspective éclatée de l'extrémité distale d'une sonde selon un premier mode de réalisation de l'invention, montrant les deux éléments essentiels de celle-ci, corps et manchon.

La figure 2 est une coupe longitudinale, selon un plan radial, du manchon.

La figure 3 illustre la cinématique de déformation du manchon au moment de la mise en place de la sonde au moment du contact avec la paroi cardiaque, respectivement pour les sections A-A et B-B de la figure 2.

Les figures 4 à 6 montrent les étapes successives de mise en place de la sonde.

La figure 7 est une vue perspective montrant la configuration de la sonde selon le premier mode de réalisation de l'invention, dans sa position finale d'ancrage.

La figure 8 illustre la manière dont peut être retiré le corps de sonde malgré la formation d'un tissu fibreux autour de celui-ci.

La figure 9 illustre une variante avantageuse de cette sonde, dans laquelle est prévue une vis de fixation.

La figure 10 est homologue de la figure 1, pour un second mode de réalisation de l'invention.

La figure 11 est une coupe longitudinale, selon un plan radial, du manchon de la sonde de la figure 10.

Les figures 12 et 13 illustrent la manière dont sont déployées les barbes de la sonde de la figure 10.

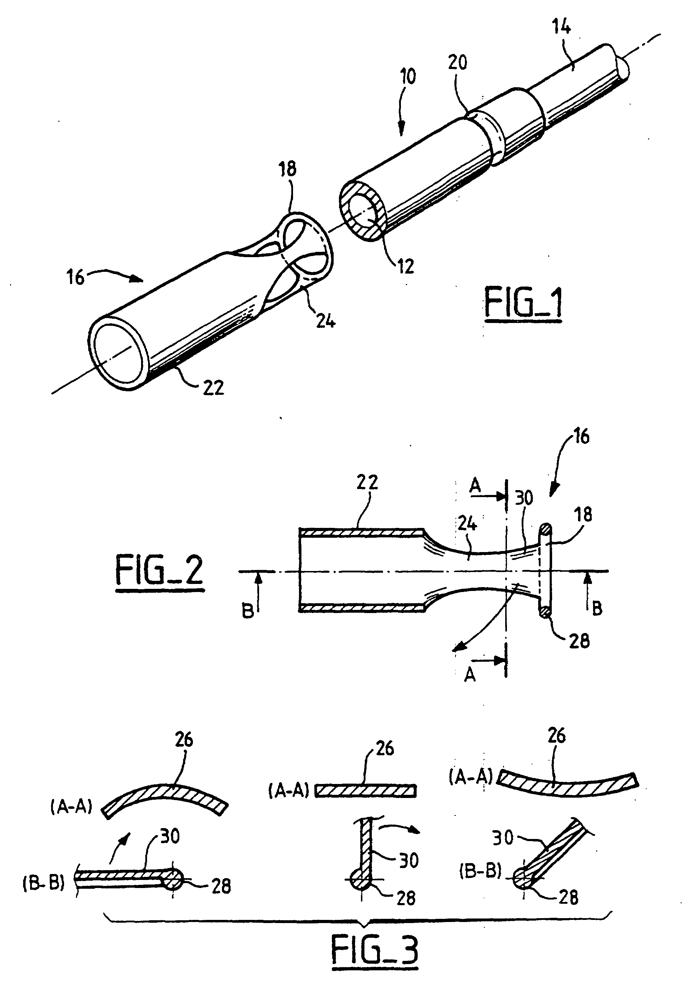

Les figures 1 à 9 se réfèrent à un premier mode de réalisation de l'invention.

Sur la figure 1, la référence 10 désigne un corps cylindrique rigide portant l'électrode de stimulation proprement dite 12 d'une sonde de stimulateur cardiaque, dont on a représenté une partie du conducteur flexible 14. L'autre extrémité de la sonde (extrémité proximale, non représentée) comporte, de manière en elle-même classique, un connecteur permettant son raccordement électrique au stimulateur distant.

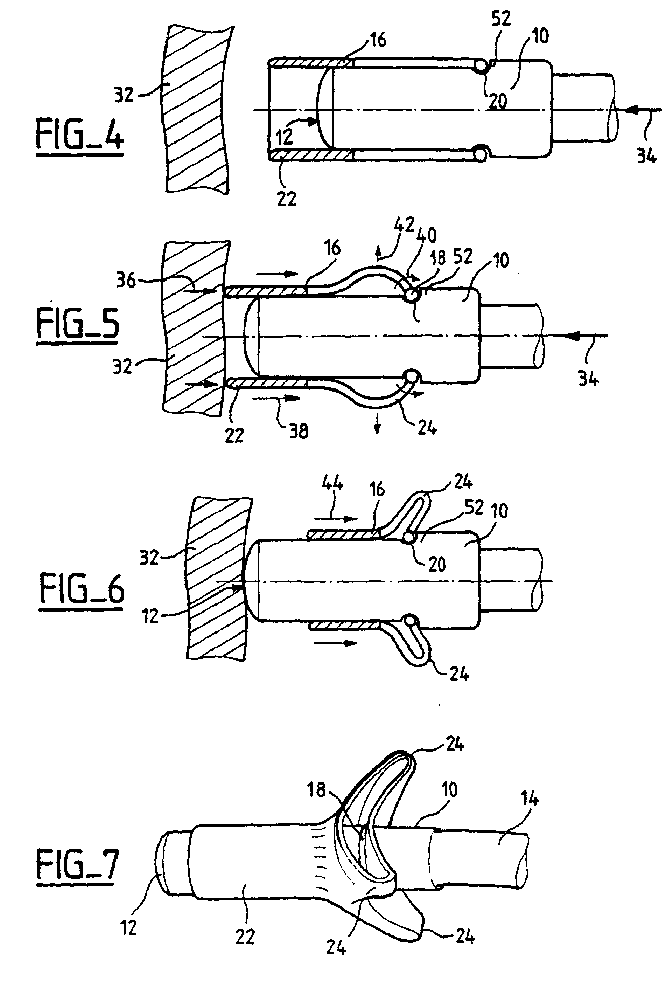

De façon caractéristique de l'invention, on prévoit un manchon cylindrique 16 qui vient coiffer le corps 10 de l'électrode (comme illustré par exemple figure 4) et réalisé par exemple en un silicone de type ETRQ7 47.80 d'une dureté Shore de l'ordre de 80, ou en un polyuréthanne de type Corethane (marque déposée) de la société Corvita. Le manchon 16 peut coulisser librement, ou avec un léger frottement (comme on l'expliquera plus bas) sur le corps 10 en direction axiale. Une bague 18, de préférence torique, vient s'encliqueter sur une gorge périphérique de section homologue 20 ménagée sur le corps 10, cette bague 18 étant reliée à la partie frontale 22 du manchon par une pluralité de ponts 24, dont on expliquera ci-dessous plus en détail le rôle. La bague 18 possède une force de striction par rapport au corps 10 évitant un détachement intempestif du manchon 16 durant l'introduction de la sonde jusqu'à la cavité cardiaque.

Comme on peut le voir sur les figures 2 et 3, les ponts 24 (qui sont sur le dessin au nombre de trois, mais ce nombre n'est aucunement limitatif) présentent une section centrale de largeur réduite avec, à l'état libre d'origine (celui illustré sur les figures 1 et 2) une concavité 26 tournée vers l'axe central du manchon (vue de gauche de la figure 3). Les extrémités proximales 30 de ces ponts, c'est-à-dire les extrémités dirigées vers le conducteur 14, sont reliées à la bague qui, du fait de sa section circulaire 28, constitue un point d'articulation autorisant une rotation de l'extrémité proximale 30 autour du centre de cette section 28, comme illustré sur les vues successives de la figure 3.

Cette rotation se fait à l'encontre de la sollicitation élastique propre du matériau du pont 24, et provoque une inversion de la concavité 26, qui maintient ainsi l'ensemble dans un autre état stable. En d'autres termes, sur la figure 3 les vues de gauche et de droite correspondent à deux états stables (stabilité résultant de la concavité des parties proximales 30, tournées, respectivement, vers l'axe du manchon de la bague et en éloignement de cet axe) et la vue centrale correspond à une position intermédiaire, instable.

Le passage d'une position à l'autre s'effectue de la manière illustrée figures 4 à 6.

Lorsque la sonde est progressivement approchée de la paroi 32 de la cavité myocardique (figure 4), le corps 10 et le manchon 16 présentent la configuration illustrée sur les figures 1 et 2 et figure 3, vue de gauche. Dans cette phase, la partie distale 22 de la sonde protège l'électrode 12 des divers frottements, par exemple contre d'autres sondes déjà installées.

Le mouvement d'approche (flèche 34) se poursuit, jusqu'à ce que l'extrémité distale du manchon vienne en contact avec la paroi 32 de la cavité (figure 5).

Une fois l'accostage ainsi réalisé, la poursuite du mouvement de progression axiale 34 de la sonde induit une force de réaction 36 sur l'extrémité distale du manchon 16. Cette réaction 36 est transmise aux ponts flexibles 24 (flèche 38) du fait de la rigidité de la partie distale 22 du manchon. Le point de flambage se localise à l'endroit présentant le moment d'inertie le plus faible, c'est-à-dire à la partie la plus rétrécie des ponts.

Ceci a pour effet de provoquer une rotation des extrémités proximales des ponts 24 autour de la bague 18 (rotation schématisée par la flèche 40 sur la figure 5) avec un écartement corrélatif radial de la partie centrale des ponts 24 (flèche 42). La situation des ponts sur la figure 5 correspond essentiellement, en section, à la vue centrale de la figure 3.

Pour éviter, par réaction, un mouvement de recul de la bague qui la ferait sortir de sa gorge 20, on prévoit avantageusement un épaulement 52 (figures 4 à 6) en arrière de cette gorge ; en d'autres termes, le corps 10 a côté proximal de la gorge un diamètre supérieur au diamètre qu'il a côté distal.

Le mouvement ainsi décrit se poursuit, jusqu'au changement brutal de concavité des ponts 24 (figure 6 et vue de droite de la figure 3) qui a pour effet, d'une part, de découvrir complètement l'électrode 12 du fait de la translation complète 44 du manchon 16 et, d'autre part, de permettre le déploiement radial complet des ponts 24, dont la partie de plus petit rayon de courbure va faire saillie radialement et avec une légère inclinaison en direction proximale, prenant ainsi la forme de barbes qui vont pouvoir venir en appui sur des trabécules de la cavité myocardique en transmettant par un effet d'action-réaction une légère pression de l'électrode 12 contre la paroi 32.

Les barbes sont ainsi constituées par les deux parties de chaque pont 24, plié au niveau de la zone centrale, à l'endroit où la section est la plus faible, comme on peut le voir très nettement sur la vue perspective de la figure 7.

Avantageusement, on prévoit de réduire le frottement du manchon 16 sur le corps 10 en plaçant un lubrifiant approprié tel qu'un hydrogel, bien entendu biocompatible, entre ces deux éléments ou sur l'un d'entre eux.

D'autre part, avantageusement, manchon et corps sont conçus de manière que, dans le mouvement de coulissement du manchon sur le corps, le frottement soit faible en début de course (positions des figures 4 et 5) puis plus important en fin de déplacement (position de la figure 6) afin de bloquer le système en position, du fait d'une résistance supplémentaire à vaincre si l'on voulait revenir à la position d'origine. En d'autres termes, on cherche à rendre le passage de la position initiale à la position finale le moins réversible possible. Ceci peut être obtenu par exemple en donnant à l'une et/ou à l'autre des pièces coopérantes une très légère conicité et/ou en prévoyant de légers reliefs sur la surface intérieure du manchon 16 et/ou sur la surface extérieure du corps 10.

On notera par ailleurs que tout déclenchement intempestif du système lors de l'introduction est empêché car les ponts 24 sont plaqués contre le corps 10 de la sonde par la sollicitation élastique des parois veineuses.

Enfin, le matériau du manchon peut avantageusement contenir des particules radio-opaques afin que le praticien puisse contrôler visuellement le bon déploiement des barbes pendant l'intervention.

Un avantage supplémentaire majeur de l'invention réside dans la facilité d'extraction de la sonde.

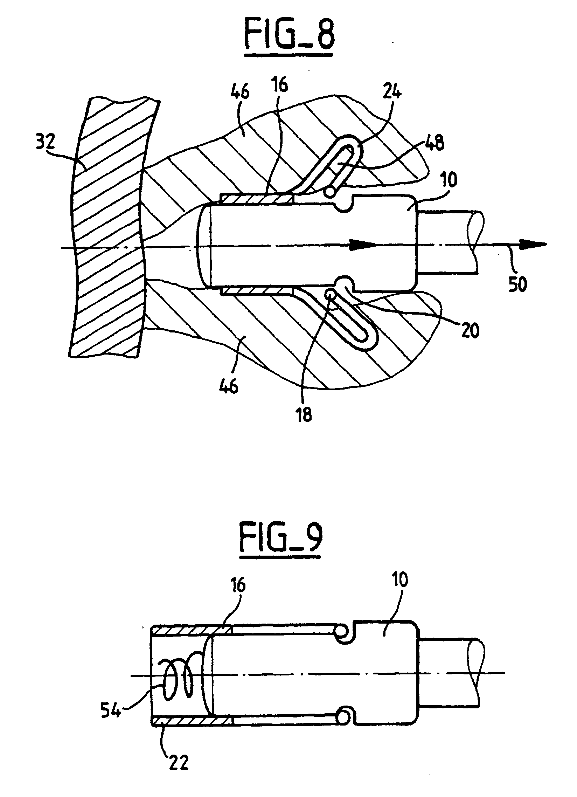

En effet, comme illustré sur la figure 8, après plusieurs mois une fibrose 46 vient généralement envelopper les barbes formées par les ponts repliés 24, et notamment le volume 48 situé entre les deux branches repliés de chaque barbe. La liaison entre barbe et fibrose est donc beaucoup plus forte qu'avec la configuration traditionnelle d'une barbe pleine, plate ou cylindrique. Dès lors, une traction 50 exercée sur le corps 10 de la sonde provoquerait quasi-immanquablement un arrachement du tissu fibreux et donc un endommagement de la paroi du myocarde.

Mais de façon avantageuse, l'anneau 18, qui est en un matériau tel que polyuréthanne ou silicone de dureté Shore 30 à 50, présente une légère élasticité autorisant une distension radiale permettant de le sortir de la gorge 20 et donc le glissement de la sonde, l'ensemble du manchon amovible restant en place, entièrement imbriqué dans la fibrose.

On peut ainsi, grâce à cette dissociation du manchon d'avec le corps, réaliser sans difficulté et sans dommage pour les tissus un retrait de la sonde par un simple mouvement de traction axiale exercé sur le conducteur.

Dans une variante avantageuse du premier mode de réalisation, illustrée figure 9, on peut pourvoir la tête de sonde d'une vis d'ancrage 54 placée sur la partie frontale de la sonde, de manière en elle-même connue. La jupe 22 joue ainsi un rôle de protecteur de la paroi veineuse au moment de l'introduction de la sonde. D'autre part, la combinaison de cette vis et du manchon selon l'invention permet d'être certain, une fois la vis ancrée à la paroi du myocarde, que les barbes ont été déployées, c'est-à-dire que le manchon se trouve dans la configuration de la figure 7.

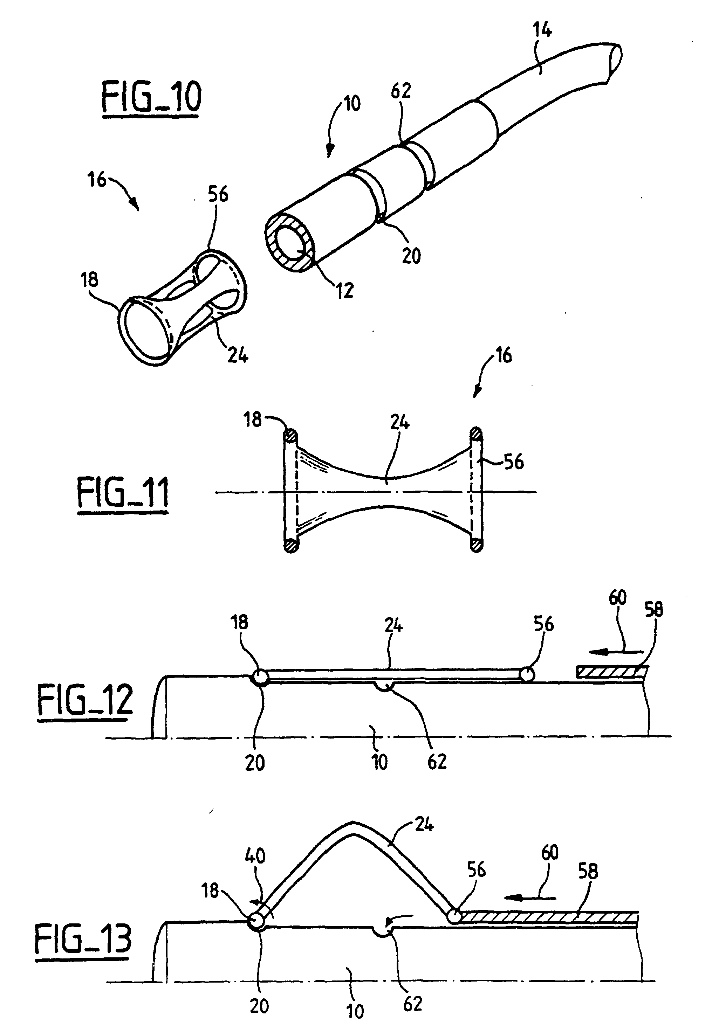

Les figures 10 à 13 illustrent un second mode de réalisation de l'invention.

Dans ce second mode de réalisation, l'élément mobile n'est plus la jupe 22, comme dans le premier mode de réalisation, mais un anneau 56 qui peut être de forme et de dimensions comparables à la bague 18, et qui est relié à celle-ci par une pluralité de ponts 24 qui jouent le même rôle que dans le premier mode de réalisation (lorsque les mêmes références numériques sont utilisées pour l'un et l'autre mode de réalisation, ces références correspondent à des éléments homologues, fonctionnellement semblables).

A l'inverse du cas précédent où l'élément mobile était situé en avant (distalement) par rapport à la bague de retenue 18, dans ce second mode de réalisation l'anneau mobile 56 est situé en arrière (proximalement) de cette même bague 18.

Dès lors, le déplacement de l'élément mobile ne se fait plus par une sollicitation frontale lors de la venue en contact de la jupe mobile avec la paroi du myocarde (c'est-à-dire une sollicitation exercée axialement sur l'extrémité distale du manchon), mais par une sollicitation arrière, exercée au moyen d'une gaine 58 (figures 12 et 13) enfilée sur le conducteur jusqu'à la sonde (c'est-à-dire une sollicitation exercée axialement sur l'extrémité proximale du manchon). La progression en direction distale ( 60 sur la figure 12) de cette gaine se poursuit jusqu'au contact de l'anneau mobile 56, qui va donc être repoussé vers l'avant (figure 13), provoquant ainsi la flexion des ponts 24. La position finale est déterminée par une gorge périphérique 62 dans laquelle vient s'emboîter définitivement l'anneau mobile 56, les ponts 24 étant alors en position de flexion complète de la même manière que cela a été explicité pour le premier mode de réalisation.

L'intervention s'achève par le retrait de la gaine 58, qui est avantageusement une gaine "pelable" à usage unique, qui peut être scindée longitudinalement en deux moitiés au fur et à mesure de son retrait

Ce second mode de réalisation présente le même avantage vis-à-vis de l'extraction que le mode de réalisation précédent, à savoir que le manchon peut se désolidariser aisément du corps de sonde pour permettre, de la même façon, l'extraction de ce dernier.

## Revendications

1. Une sonde pour dispositif médical implanté, notamment pour stimulateur cardiaque, comportant un corps cylindrique (10) portant l'électrode (12) ou les électrodes de la sonde,
**caractérisée en ce qu'**elle comprend en outre un manchon amovible (16) coiffant le corps d'électrode, ce manchon comportant :
- une bague (18) de retenue du manchon sur le corps, le montage de cette bague sur le corps étant amovible de manière à permettre la dissociation ultérieure du manchon d'avec le corps par traction axiale de ce dernier, et
- des moyens déformables (24) s'étendant à partir de la bague de retenue et susceptibles de prendre deux configurations stables, le passage de la première à la seconde configuration résultant d'une sollicitation extérieure (36 ; 58) exercée axialement sur le manchon, ces moyens ne faisant essentiellement pas saillie radialement dans la première configuration, de manière à permettre l'introduction en place de la sonde, et faisant essentiellement saillie radialement dans la seconde configuration de manière à former des barbes d'ancrage.

2. La sonde de la revendication 1, dans laquelle les barbes d'ancrage des moyens déformables sont reliées entre elles par un élément mobile (22 ; 56) entourant le corps et coulissant le long de celui-ci entre une position initiale et une position finale, ces deux positions correspondant respectivement aux deux configurations stables des moyens déformables, ces moyens déformables formant moyens de liaison entre la bague de retenue (18) et l'élément mobile (22; 56).

3. La sonde de la revendication 1, dans laquelle l'élément mobile est une jupe (22) placée en position distale par rapport à la bague (18), cette jupe faisant axialement saillie du corps en position initiale et n'en faisant pas saillie en position finale, et le passage de la première à la seconde configuration résultant de la sollicitation extérieure (36) exercée axialement dans le sens proximal sur l'extrémité distale de la jupe lors de l'accostage de celle-ci avec une paroi (32) contre laquelle doit venir en contact l'électrode.

4. La sonde de la revendication 3, comprenant des moyens pour augmenter le frottement entre corps et jupe au voisinage de la position finale.

5. La sonde de la revendication 4, dans laquelle, pour augmenter le frottement, au voisinage de la position finale le corps présente un diamètre extérieur accru et/ou la jupe présente un diamètre intérieur accru.

6. La sonde de la revendication 4, dans laquelle, pour augmenter le frottement, au voisinage de la position finale le corps présente des reliefs sur sa surface extérieure et/ou la jupe présente des reliefs sur sa surface intérieure.

7. La sonde de la revendication 3, dans laquelle le corps présente un épaulement de butée (52) en arrière de la position finale.

8. La sonde de la revendication 3, comprenant en outre sur la face frontale du corps une vis d'ancrage (54), cette vis étant recouverte par la jupe en position initiale et en faisant saillie axialement en position finale.

9. La sonde de la revendication 2, dans laquelle l'élément mobile est un anneau (56) placé en position proximale par rapport à la bague (18), et le passage de la première à la seconde configuration résultant de la sollicitation extérieure (60) exercée axialement dans le sens distal sur l'anneau par manipulation d'une gaine (58) enfilée sur le corps.

10. La sonde de la revendication 9, dans laquelle le corps porte une gorge périphérique de blocage (62) recevant et immobilisant l'anneau en fin de course.

11. La sonde de la revendication 2, dans laquelle les moyens de liaison comprennent une pluralité de ponts (24) en un matériau élastiquement déformable.

12. La sonde de la revendication 11, dans laquelle les ponts ont en section un profil concave, cette concavité s'inversant entre la première et la seconde configuration.

13. La sonde de la revendication 2, dans laquelle la bague de retenue (18) a une forme essentiellement torique et le corps cylindrique possède une gorge périphérique (20) de section circulaire recevant cette bague torique, gorge et tore ayant des diamètres homologues sensiblement identiques de manière à permettre en tout point le pivotement local de la bague dans la gorge.

## Claims

1. A probe for an implanted medical device, in particular a pacemaker, the probe comprising a cylindrical body (10) carrying the electrode (12) or the electrodes of the probe,
the probe being **characterized in that** it further comprises a removable sleeve (16) covering the electrode body, said sleeve comprising:
- a retaining ring (18) for holding the sleeve on the body, said ring being assembled on the body in removable manner so as to enable the sleeve subsequently to be separated from the body by applying axial traction thereto; and
- deformable means (24) extending from the retaining ring and suitable for taking two stable configurations, transition from the first configuration to the second resulting from an external force (36; 58) exerted axially on the sleeve, said means essentially not projecting radially while in the first configuration so as to enable the probe to be put into place, and essentially projecting radially when in the second configuration so as to form anchoring barbs.

2. The probe of claim 1, in which the anchoring barbs of the deformable means are interconnected by a moving element (22; 56) surrounding the body and sliding therealong between an initial position and a final position, these two positions corresponding respectively to the two stable configurations of the deformable means, said deformable means forming link means between the retaining ring (18) and the moving element (22; 56).

3. The probe of claim 1, in which the moving element is a skirt (22) placed in a distal position relative to the ring (18), said skirt projecting axially from the body in the initial position and not projecting in the final position, and the transition from the first configuration to the second configuration resulting from the external force (36) that is exerted axially in the proximal direction on the distal end of the skirt while it is docking with a wall (32) against which the electrode is to come into contact.

4. The probe of claim 3, including means for increasing friction between the body and the skirt in the vicinity of the final position.

5. The probe of claim 4, in which in order to increase friction in the vicinity of the final position, the body presents an increased outside diameter and/or the skirt presents an increased inside diameter.

6. The probe of claim 4, in which in order to increase friction in the vicinity of the final position, the body presents portions in relief on its outside surface and/or the skirt presents portions in relief on its inside surface.

7. The probe of claim 3, in which the body presents an abutment shoulder (52) behind the final position.

8. The probe of claim 3, further comprising, on the end face of the body, an anchor screw (54), said screw being covered by the skirt in the initial position and projecting axially in the final position.

9. The probe of claim 2, in which the moving element is an annulus (56) placed in the proximal position relative to the ring (18), and the transition from the first configuration to the second configuration results from the external force (60) exerted axially in the distal direction on the annulus by handling a sheath (58) engaged on the body.

10. The probe of claim 9, in which the body carries a peripheral locking groove (62) receiving the annulus at the end of its stroke and preventing it from moving.

11. The probe of claim 2, in which the link means comprise a plurality of bridges (24) of elastically deformable material.

12. The probe of claim 11, in which the bridges are of section that is concave in profile, the concave side changing sides between the first configuration and the second configuration.

13. The probe of claim 2, in which the retaining ring (18) is essentially toroidal in shape and the cylindrical body possesses a peripheral groove (20) of circular section receiving said toroidal ring, the groove and the torus having corresponding diameters that are essentially identical so as to enable the ring to pivot locally at any point in the groove.

## Patentansprüche

1. Sonde für implantierte medizinische Vorrichtung, insbesondere für Herzschrittmacher, aufweisend einen zylindrischen Körper (10), der die Elektrode (12) oder die Elektroden der Sonde trägt,
**dadurch gekennzeichnet, dass**
sie des Weiteren eine lösbare Hülse (16) aufweist, welche den Körper der Elektrode bedeckt, wobei die Hülse aufweist:
- einen Rückhaltering (18) der Hülse auf dem Körper, wobei die Montage des Rings auf dem Körper lösbar ist, um die spätere Trennung der Hülse von dem Körper durch axialen Zug des letzteren zu erlauben, und
- deformierbare Mittel (24), die sich von dem Rückhaltering erstrekken und geeignet sind, zwei stabile Konfigurationen anzunehmen, wobei der Übergang von der ersten zu der zweiten Konfiguration aus einer äußeren Beanspruchung (36; 58) resultiert, welche axial auf die Hülse ausgeübt wird, wobei die Mittel nicht wesentlich radial überstehen in der ersten Konfiguration, um die Einführung der Sonde an den Platz zu erlauben, und wesentlich radial überstehen in der zweiten Konfiguration, um Verankerungshaken zu bilden.

2. Sonde gemäß Anspruch 1, bei welcher die Verankerungshaken der deformierbaren Mittel untereinander verbunden sind durch ein mobiles Element (22; 56), welches den Körper umgibt und entlang desselben gleitet zwischen einer Anfangsposition und einer Endposition, wobei die zwei Positionen jeweils den zwei stabilen Konfigurationen der deformierbaren Mittel entsprechen, wobei die deformierbaren Mittel Verbindungsmittel zwischen dem Rückhaltering (18) und dem mobilen Element (22; 56) bilden.

3. Sonde gemäß Anspruch 1, bei welcher das mobile Element ein Mantel (22) ist, der in distaler Position mit Bezug auf den Ring (18) platziert ist, wobei der Mantel axial vorsteht von dem Körper in Anfangsposition und nicht vorsteht in Endposition, wobei der Übergang von der ersten zu der zweiten Konfiguration aus der äußeren Beanspruchung (36) resultiert, welche axial in proximaler Richtung ausgeübt wird auf das distale Ende des Mantels während des Ankoppelns desselben mit einer Wand (32), gegen welche die Elektrode in Kontakt kommen muss.

4. Sonde gemäß Anspruch 3, Mittel aufweisend zum Erhöhen der Reibung zwischen Körper und Mantel in der Nähe der Endposition.

5. Sonde gemäß Anspruch 4, bei welcher, um die Reibung zu erhöhen, in der Nähe der Endposition der Körper einen anwachsenden äußeren Durchmesser aufweist und/oder der Mantel einen anwachsenden inneren Durchmesser aufweist.

6. Sonde gemäß Anspruch 4, bei welcher, um die Reibung zu erhöhen, in der Nähe der Endposition der Körper Reliefs auf seiner äußeren Oberfläche aufweist und/oder der Mantel Reliefs auf seiner inneren Oberfläche aufweist.

7. Sonde gemäß Anspruch 3, bei welcher der Körper eine Anschlagschulter (52) hinter der Endposition aufweist.

8. Sonde gemäß Anspruch 3, des Weiteren aufweisend auf der frontalen Fläche des Körpers eine Verankerungsschraube (54), wobei die Schraube bedeckt ist durch den Mantel in Anfangsposition und axial vorsteht in Endposition.

9. Sonde gemäß Anspruch 2, bei welcher das mobile Element ein Ring (56) ist, der in proximaler Position in Bezug zu dem Ring (18) platziert ist, und der Übergang von der ersten zu der zweiten Konfiguration aus der äußeren Beanspruchung (60) resultiert, welche axial in distaler Richtung ausgeübt wird auf den Ring durch Handhabung einer Hülse (58), die auf den Körper aufgebracht wird.

10. Sonde gemäß Anspruch 9, bei welcher der Körper eine Einrast-Umfangskehle (62) trägt, welche den Ring am Laufende aufnimmt und immobilisiert.

11. Sonde gemäß Anspruch 2, bei welcher die Bindungsmittel eine Mehrzahl von Brücken (24) aus einem elastisch deformierbaren Werkstoff aufweisen.

12. Sonde gemäß Anspruch 11, bei welcher die Stege im Schnitt ein konkaves Profil aufweisen, wobei diese Konkavität sich umkehrt zwischen der ersten und der zweiten Konfiguration.

13. Sonde gemäß Anspruch 2, bei welcher der Rückhaltering (18) eine im Wesentlichen torusförmige Form aufweist und der zylindrische Körper eine Umfangsnut (20) von kreisförmigem Querschnitt aufweist, welche den torusförmigen Ring aufnimmt, wobei Kehle und Torus entsprechende Durchmesser aufweisen, die im Wesentlichen identisch sind, um in jedem Punkt das lokale Drehen des Rings in der Kehle zu erlauben.
